# EUROPEAN PATENT SPECIFICATION

(11) **EP 3 634 458 B1**
(45) Date of publication and mention of the grant of the patent: **06.08.2025**
(21) Application number: 18729492.1
(22) Date of filing: 10.05.2018
(51) Int. Cl.: A61K 38/00, A61K 31/19, A61P 1/00

(54) **COMPOSITION TO PREVENT INTESTINAL DAMAGE**
ZUSAMMENSETZUNG ZUR PRÄVENTION VON DARMSCHÄDEN
COMPOSITION DESTINÉE À PRÉVENIR LES LÉSIONS INTESTINALES

(30) Priority: 10.05.2017 IT 201700050442
(43) Date of publication of application: 15.04.2020
(73) Proprietor: Fusion Farm S.r.l. Semplificata, 01100 Viterbo (IT)
(72) Inventor: VOLPARI, Dario, 20148 Milano (IT); TOCCHI, Grazia, 01100 Viterbo (IT)
(74) Representative: Biggi, Cristina
(86) International application number: PCT/IB2018/053248
(87) International publication number: WO 2018/207122

(56) References cited:
- WO-A1-2008/147228
- WO-A2-2008/140335
- WO-A2-2009/052106
- L. PENG ET AL: "Butyrate Enhances the Intestinal Barrier by Facilitating Tight Junction Assembly via Activation of AMP-Activated Protein Kinase in Caco-2 Cell Monolayers", THE JOURNAL OF NUTRITION, vol. 139, no. 9, 22 July 2009 (2009-07-22), US, pages 1619 - 1625, XP055374908, ISSN: 0022-3166, DOI: 10.3945/jn.109.104638
- TIROTTA ERIKA ET AL: "Enteric Protective Effects of the CombinationBifidobacteriumLongum and Lactoferrin in a Rat Model of Diclofenac-Induced Intestinal Injury", GASTROENTEROLOGY, W.B. SAUNDERS CO, US, 22 April 2017 (2017-04-22), XP085106036, ISSN: 0016-5085, DOI: 10.1016/S0016-5085(17)31614-1
- BELINDA VAN'T LAND ET AL: "Lactoferrin Reduces Methotrexate-Induced Small Intestinal Damage, Possibly Through Inhibition of GLP-2-Mediated Epithelial Cell Proliferation", DIGESTIVE DISEASES AND SCIENCES, vol. 49, no. 3, 1 March 2004 (2004-03-01), pages 425 - 433, XP055010171, ISSN: 0163-2116, DOI: 10.1023/B:DDAS.0000020497.35250.93

## Description

### FIELD OF THE INVENTION

The invention concerns a composition for the prevention of intestinal damage, preferably resulting from villous atrophy or loss of integrity of the intestinal barrier.

### STATE OF THE ART

A good part of intestinal diseases is mainly due to malabsorption of nutrients due to villous atrophy in the gastrointestinal tract epithelium, or even to an alteration at the cellular junctions level. In these cases, there is a significant damage to the intestinal mucosal epithelium which is no longer able to correctly perform its barrier function, being no longer able to correctly filter and absorb the nutrients needed by the organism.

This kind of diseases are mainly found in patients undergoing prolonged parenteral nutrition, in malnutrition, in celiac disease, in the elderly, in mucositis induced by chemotherapeutic agents (for example, methotrexate), in immunodeficiencies, or in genetic or immune alterations also responsible for dysfunctions of the intestinal barrier. Similar diseases, always due to malabsorption of nutrients, are also widely found in animals.

To treat these diseases, many types of products are currently used.

For example, products containing probiotics, i.e. live organisms promoting intestinal well-being, such as, among the most common, lactobacilli and bifidobacteria, also known as "lactic ferments", which are formulated both as pharmaceuticals and as supplements.

The use of products containing prebiotics, which represent the nourishment of probiotics stimulating their activity in the gastrointestinal tract, individually or in combination with lactobacilli, or even the use of products containing nucleotides, used in recent years as supplements and nutritional supplements for their nutraceutical and immunostimulant properties, is also known.

Many scientific works about intestinal diseases and possible molecules that may be used for their treatment have been carried out and published.

In the recent and interesting scientific review by Annaïg Lan et Al (A. Lan, F. Blachier, R. Benamouzig, M. Beaumont, C. Barrat, D. Coelho, A. Lancha, X. Kong, Y. Yin, J.C. Marie, e D. Tomé, "Mucosal healing in inflammatory bowel diseases: is there a place for nutritional supplementation?" Review Article in Inflammatory Bowel Diseases, September 2014) for example, the clinical and preclinical studies performed over time to evaluate the chance of mucosal healing in intestinal diseases, thanks to the use of various types of nutritional supplements, such as amino acids (glutamine, arginine, glutamate, threonine, serine, proline, methionine), short-chain fatty acids, lipids, vitamins (A, D3, C), and minerals (zinc), are reviewed. The study concludes by observing that many of these compounds exhibit some positive activities at the intestinal level, but their mechanisms of action and the understanding of the dynamics of biological interaction are far from known.

Among the many active agents that may be used to treat intestinal diseases, butyric acid is also mentioned. For example, esters and/or salts thereof are present in many dietary supplements for the treatment of patients affected by colonopathies with altered intestinal mucosal trophism.

It is, in fact, known that butyric acid is the preferred source of energy for colonocytes and plays a key role in maintaining the integrity of the mucosa, and in the intestinal lesions repair processes, by stimulating water and sodium reabsorption in the colon, contributing to lower the intestinal pH, and thus creating an unfavorable environment for the development of pathogenic bacteria.

Another ingredient sometimes used to combat intestinal diseases is lactoferrin. It is, in fact, known that lactoferrin has anti-infective, immunomodulatory properties and promotes a correct intestinal ecology. It is an antimicrobial and iron-carrying glycoprotein, and its antimicrobial properties are mainly due to the ability to bind iron, thus subtracting it from the metabolism of the bacterial species that are dependent on it for their multiplication and adhesion to the intestinal mucosa.

WO 2008/140335 discloses synergistic compositions of milk fat comprising butyric acid and lactoferrin for use in treating intestinal damage.

Despite the great variety of treatments used against intestinal disorders, the need for alternative therapies is, however, always strongly felt, also considering the subjective patients' response to the various treatments.

In addition, to date, there is no known treatment that may be used in the prevention of intestinal epithelium tissue damage, particularly frequent for certain patients suffering from various diseases, or treated with drugs causing tissue damage.

The object of the present invention is therefore to prevent tissue damage, preferably resulting from villous atrophy or loss of intestinal barrier integrity.

### SUMMARY OF THE INVENTION

The inventors have surprisingly found that the combination of lactoferrin and a salt of butyric acid has positive effects on the intestinal mucosa, wherein the salt of butyric acid is sodium butyrate and wherein the weight ratio between sodium butyrate amount and lactoferrin amount is greater than or equal to 2, improving the barrier function at the level of the intracellular junction protein expression, and counteracting villous atrophy.

Such combination surprisingly prevents tissue damage that may occur in certain patients, for example, in patients undergoing prolonged parenteral nutrition, in celiac patients, in the elderly, in patients with mucositis induced by chemotherapeutic agents (for example, methotrexate), in patients with alterations of the intestinal barrier due to malnutrition and/or immunodeficiency, or due to genetic and immune disorders.

Such combination, preventing tissue damage, may also be used in animals in case of malabsorption syndromes resulting also from extra-intestinal diseases of various kinds.

The invention therefore concerns a composition comprising a salt of butyric acid, and lactoferrin, wherein the salt of butyric acid is sodium butyrate and wherein the weight ratio between sodium butyrate amount and lactoferrin amount is greater than or equal to 2.

In another aspect, the invention relates to a pharmaceutical form comprising the composition of the invention and at least one pharmacologically acceptable excipient.

Said pharmaceutical form is preferably selected from the group consisting of a granulate, a powder, a syrup, a tablet, a capsule, a gel, an aqueous suspension, a liquid.

Preferably, said pharmaceutical form comprising the composition of the invention is a powder, a granulate, or an aqueous suspension, advantageously and preferably provided in a single-dose sachet format for oral administration. In another aspect, the invention concerns the composition or the pharmaceutical form comprising said composition for use in the prevention of villous atrophy typical of the elderly patients.

Furthermore, the inventors have surprisingly discovered that the solubility of lactoferrin in aqueous solution was significantly improved when it was used in combination with sodium butyrate. The composition of the invention resulted therefore advantageous, since the combined use of sodium butyrate with lactoferrin has allowed an excellent solubilization of lactoferrin in water.

### DESCRIPTION OF THE FIGURES

Figure 1 shows the flux percentage of the substance Lucifer Yellow (LY) in presence (PC) of intestinal epithelium damage (40 h in glutamine free medium followed by stress induced by 60 mM ethanol for 4 h) compared with negative control (NC).
Figure 2 shows the results obtained by fluorescence immunolabeling of Occludin and Zonula Occludens in Caco-2 cells after epithelial damage.
Figure 3 shows the results obtained by SEM microscopy in Caco-2 cells before (Figure 3A) and after epithelial damage (Figure 3B).
Figure 4 shows the percentage of the substance Lucifer Yellow (LY) after 60 hours of glutamine deprivation in the absence (PC) or in the presence of the products A/B/C, where A=2 mM sodium butyrate, B=400 µg/ml lactoferrin, and C=2 mM sodium butyrate + lactoferrin 400 µg/ml.
Figure 5 shows the results obtained by fluorescence immunolabeling of Occludin and Zonula Occludens in Caco-2 cells in the absence (PC) or after pretreatment with the products A/B/C, where A=2 mM sodium butyrate, B=400 µg/ml lactoferrin, and C=2 mM sodium butyrate + lactoferrin 400 µg/ml.
Figure 6 shows the results obtained by SEM microscopy in Caco-2 cells after pretreatment with the products A/B/C, where A=2 mM sodium butyrate, B=400 µg/ml lactoferrin, and C=2 mM sodium butyrate + lactoferrin 400 µg/ml.

### DETAILED DESCRIPTION OF THE INVENTION

The invention therefore concerns a composition comprising lactoferrin and a salt of butyric acid, wherein the salt of butyric acid is sodium butyrate and wherein the weight ratio between sodium butyrate amount and lactoferrin amount is greater than or equal to 2.

In the present invention, when using the term:
- "salt", it means a salt, preferably a pharmaceutically acceptable salt, obtained by salification of butyric acid with metals of the first and second group, such as for example sodium or calcium butyrate.
- "ester", it means an ester, preferably a pharmaceutically acceptable ester, such as for example methyl butyrate, ethyl butyrate, triglyceryl tributyrate.

The composition of the invention comprises lactoferrin and a pharmaceutically acceptable salt of butyric acid, i.e.sodium butyrate.

Said composition comprises a weight ratio between sodium butyrate amount and lactoferrin amount greater than or equal to 2, preferably in the range of about 2 to 5.

The composition of the invention may further comprise at least one pharmaceutically acceptable excipient suitable for its pharmaceutical formulation, such as for example microcrystalline cellulose, gum arabic, corn starch, mannitol, xylitol, glucose, sucrose, saccharin, aspartame, lemon essential oil, talc, stearic acid, gelatin, glycerol, propylene glycol, sorbitol, PVP, carboxymethylcellulose, hydroxyethylcellulose, methylcellulose, polyvinyl alcohol, shellac, cellulose acetophthalate, hydroxypropylmethylcellulose.

In another aspect, the invention therefore relates to a pharmaceutical form comprising the composition of the invention and at least one pharmaceutically acceptable excipient.

Preferably, said pharmaceutical form is in the form of a supplement or food supplement.

Preferably said pharmaceutical form comprising the composition of the invention comprises a unitary dosage of equivalent to an amount of sodium butyrate in the range of 2 mg to 5 g, more preferably in an amount comprised in the range of 10 mg to 1 g, even more preferably said composition of the invention comprises a unitary dosage of sodium butyrate in the range of 450 mg to 750 mg.

Alternatively, said pharmaceutical form comprising the composition of the invention comprises lactoferrin in a unitary dosage in the range of 5 mg to 5 g, more preferably in a unitary dosage in the range of 100 mg to 2 g, even more preferably said composition of the invention comprises lactoferrin in a unitary dosage in the range of 150 mg to 350 mg.

In a further preferred embodiment of the invention, said pharmaceutical form comprising the composition of the invention comprises a unitary dosage of sodium butyrate in an amount in the range of 10 mg to 1 g and a unitary dosage of lactoferrin in the range of 100 mg to 2000 mg, preferably it comprises a unitary dosage of sodium butyrate in an amount in the range of 450 mg to 750 mg and a unit dose of lactoferrin in an amount in the range of 150 mg to 350 mg.

In yet another preferred embodiment of the invention, said pharmaceutical form comprises a weight ratio between the unitary dosage of sodium butyrate and the unitary dosage of lactoferrin greater than or equal to 2, preferably in the range of about 2 to about 5.

Preferably, said pharmaceutical form is for oral use, preferably selected from the group consisting of a granulate, a powder, a syrup, a tablet, a capsule, a gel, an aqueous suspension, and a liquid.

In a preferred embodiment, said pharmaceutical form is a powder.

In a further preferred embodiment, said pharmaceutical form is a granulate.

In a further preferred embodiment, said pharmaceutical form is an aqueous suspension.

Advantageously, when the pharmaceutical form is selected from the group consisting of a powder, a granulate, a suspension, it is in the form of a sachet, preferably a single-dose sachet for oral administration.

In another aspect, the invention concerns the composition or the pharmaceutical form comprising said composition for the use in the prevention of villous atrophy typical of elderly patients.

Without being tied to any theory, the inventors believe that the composition of the invention is able to prevent intestinal damage thanks to the synergy of its components.

Specifically, the composition of the invention, preferably in the pharmaceutical form of a powder, even more preferably diluted in water, once taken reaches the gastrointestinal mucosa, where butyric acid, which plays a key role in maintaining the integrity of the mucosa and in intestinal lesions repair processes, being able to positively influence the very low solubility of lactoferrin, and therefore allowing an increased bioavailability, would make it better able to perform its anti-infective, immunomodulatory properties and promote a correct intestinal ecology.

The composition of the invention, as will be apparent from the following experimental part hereinafter, prevents in fact intestinal damage.

Said composition, by preventing damage to the intestinal epithelium, maintains the physiological functionality of the gastrointestinal mucosa, counteracting the process of villous atrophization and the loss of barrier integrity.

Disclosed herein is the composition or the pharmaceutical form for use in the prevention of intestinal damage in patients undergoing prolonged parenteral nutrition, in patients with genetic predisposition to celiac disease, in patients receiving chemotherapeutic agents, or in patients having genetic or immune disorders responsible also for the alteration of the intestinal barrier, or in the prevention of the villous atrophy typical of the elderly patients.

The composition of the invention, which comprises lactoferrin and sodium butyrate, resulted to be surprisingly advantageous in combating intestinal villous atrophy, and is able to decrease the membrane permeability thanks to recovery of barrier integrity.

As it will be apparent from the experimental part that follows, the inventors have surprisingly discovered that using in combination lactoferrin and sodium butyrate, wherein the weight ratio between sodium butyrate amount and lactoferrin amount is greater than or equal to 2, on an intestinal mucosa on which a stress was induced, a barrier integrity maintenance, at the level of the intestinal epithelium intracellular junctions and at the level of intestinal villous atrophy, evidence of efficacy of the composition of the invention, in the prevention of intestinal mucosal damage, was observed.

As it will also be apparent from the experimental part that follows, the solubility of lactoferrin in aqueous solution resulted to be surprisingly improved, when in combination with sodium butyrate. The composition of the invention was therefore further advantageous since the combined use of sodium butyrate and lactoferrin also allowed a better solubilization of lactoferrin in water.

### Experimental Part

### Example 1

In order to demonstrate that the composition of the invention prevents intestinal damage, a model of intestinal damage was developed based on the induction of morphological changes of the microvilli and the barrier function, and the efficacy of the combination of sodium butyrate and lactoferrin was evaluated.

A protocol was developed based on Caco-2 cell inserts model (CACOREADY^{™} cells, lot no. 24 676 101716 and no. 24 680 111416, Readycell manufacturer) consisting in two steps:
1. Induction of epithelial damage (40 h incubation in glutamine free medium followed by stress induced by 60 mM ethanol for 4 h)
2. Evaluation of the single products first, and then applied in combination during stress induced by the absence of glutamine (60 h), and with subsequent stress induced by ethanol, to evaluate the efficacy in preventing damage.

### 1.1. Model of Intestinal Epithelial Damage: Damage Induction.

Caco-2 cells were incubated for 40 hours in glutamine free medium, followed by a phase of treatment of the same cells with 60 mM ethanol for 4 hours in order to induce stress (PC= with damage).

Cells without damage induction (NC=without damage) and with damage induction (PC) were evaluated with the Lucifer Yellow (LY) assay, which verifies the integrity of the cellular junctions in presence of the test substance. LY is a fluorescent marker impermeable to the cell membrane, and it is used to study the permeability of a substance at the paracellular level.

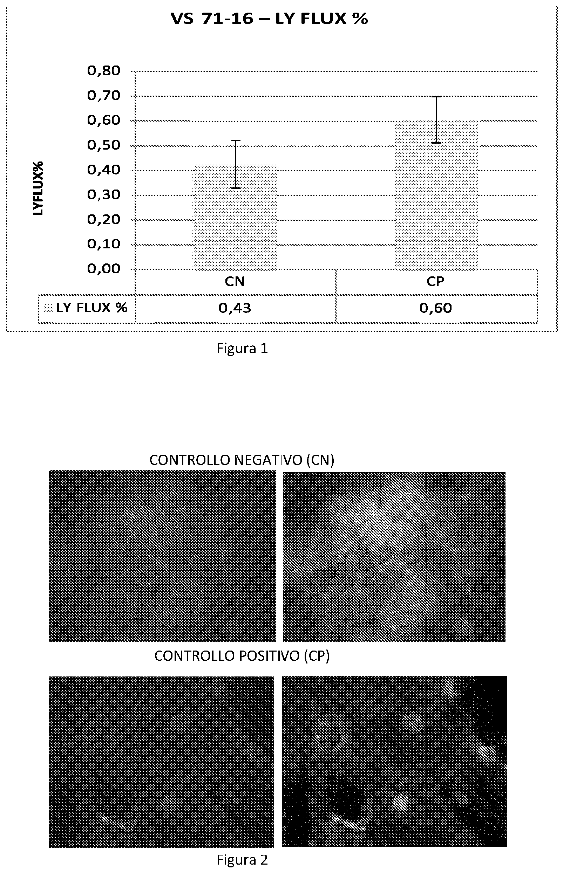

The results are shown in Figure 1: on the left side LY percentage values of cells without damage (NC) are shown, and on the right side the results of the cells following damage (PC) are shown.

As it can be seen by Figure 1, the damage induced an increase in permeability. The same cells without damage induction (NC) and with damage induction (PC), as indicated above, were further evaluated by fluorescence immunolabeling of Occludina and Zonula Occludens (proteins present in the intestinal membrane intracellular junctions).

Figure 2 shows the images of the cells: the negative control without damage (NC) in the upper part, and the cells with damage induction (PC) in the lower part.

As it can be seen from Figure 2, the intestinal mucosa of cells with damage (PC) was damaged. Specifically, holes were detected among the epithelial cells and the labeling of both proteins was less intense.

The same cells without damage induction (NC) and with damage induction (PC), as indicated above, were finally evaluated by Electron Microscopy (SEM) at different magnifications.

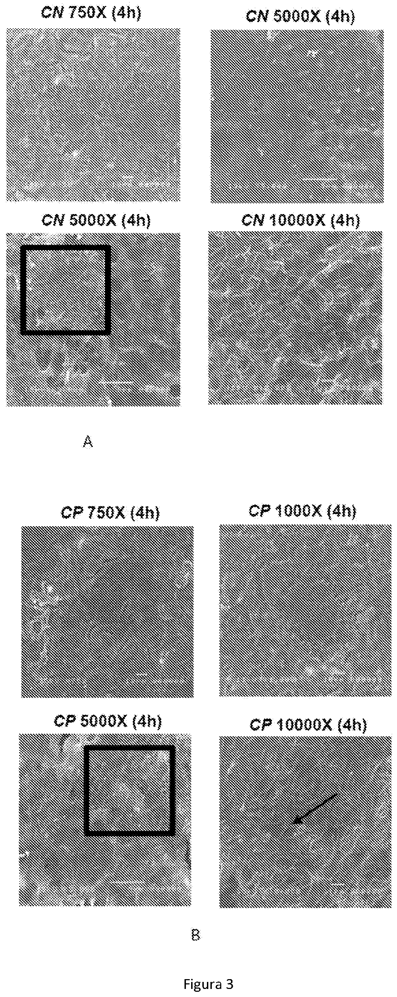

The results are shown in Figure 3A for cells without damage (NC) and in Figure 3B for cells with damage (PC).

In Figure 3A, it can be seen that the microvilli distribution on the cell surface appeared homogeneous, with microvilli that appeared arborescent and well-structured with alpha-actinin caps (typical of the microvilli head), structured in macroaggregates (magnification 5000X with the box visible also at 10000x).

In Figure 3B, showing cells with damage (PC), it can be seen that the microvilli distribution on the cell surface appeared homogeneous (5000x), but the microvilli did not appear arborescent and were flat on the cellular surface (magnification 5000X with the box visible also at 10000x), losing the upright position and the three-dimensionality.

### 1.2. Evaluation of the Efficacy of the Composition of the Invention in Damage Prevention.

According to the experimental protocol, to evaluate the efficacy of the composition of the invention in damage prevention, sodium butyrate (2 mM concentration) and lactoferrin (400 µg/ml concentration), either alone or in combination, were applied on Caco-2 cells during stress induced by absence of glutamine, increasing the time of said deprivation, with respect to the conditions tested during the development of the model, and therefore of stress induction, to 60 hours, to make more evident differences in cells behavior in the different treatment situations. At the end of 60 hours, the products were removed, and damage was induced for 4h with 60 mM ethanol. The parameters were evaluated at the end.

### 1.2.1. Evaluation of the Cells by Lucifer Yellow (LY) Assay.

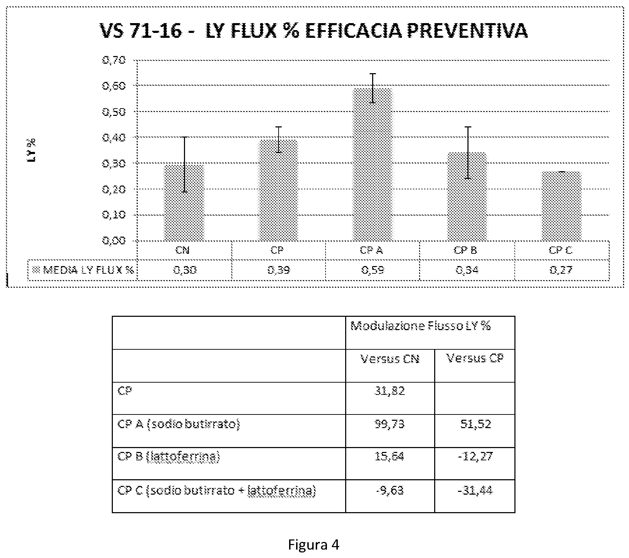

The results of the assay with LY, shown in Figure 4, show the percentage of the substance Lucifer Yellow (LY) after 60 hours of glutamine deprivation (PC) and in the presence of the products A/B/C, where A=2 mM sodium butyrate, B=400 µg/ml lactoferrin and C=2 mM sodium butyrate + 400 µg/ml lactoferrin.

As it can be seen by Figure 4, the treatment (C) with the combination of 2 mM sodium butyrate and 400 µg/ml lactoferrin, applied during damage induction, was able to generate over 31% reduction in LY flux with respect to positive control (PC).

Surprising result, observing that the application of lactoferrin alone was able to induce only a modest improvement of cell conditions, with about 12% reduction in LY flux, while even the addition of sodium butyrate alone caused a significant worsening of the barrier integrity, highlighting an increase in LY flux, and therefore of the permeability, of even over 51%.

Therefore, sodium butyrate and lactoferrin, used in combination, showed a surprisingly positive and synergic behavior in the prevention of barrier damage. Not only that, from the observation of the numerical data obtained and set out in the table in Figure 4, it also emerges that the synergy of action of the two substances in the composition of the invention allowed an improvement of the general state of health even in healthy cell, not subjected to damage induction; the comparison versus negative control (NC) showed, in fact, a marked improvement in the barrier integrity, consistently with the measurement of a decrease of about 10% in LY flux.

### 1.2.2. Evaluation of the Cells by Fluorescence Immunolabeling of Occludin and Zonula Occludens Investigation

To confirm the observed behavior, an evaluation using fluorescence immunolabeling of Occludin and Zonula Occludens was also performed.

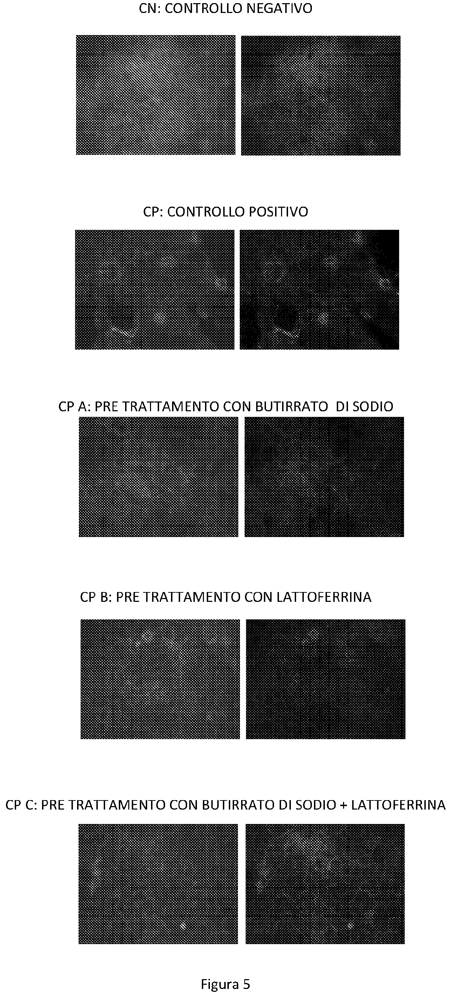

Figure 5 shows images, obtained by imunolabeling, of the negative control cells (NC) and cells in the absence (PC) or after pretreatment with the products A/B/C (PC A, PC B, PC C), where A=2 mM sodium butyrate, B= 400 µg/ml lactoferrin and C= 2 mM sodium butyrate + 400 µg/ml lactoferrin.

Also from the observation of these images, it was evident that the combination of butyrate and lactoferrin had a protective effect against damage induction, highlighted both by a recovery of the fluorescence intensity and the barrier integrity.

### 1.2.3. Evaluation of the Cells by Electron Microscopy (SEM) Investigation.

The same cells were finally evaluated also by Electron Microscopy SEM, at various magnifications.

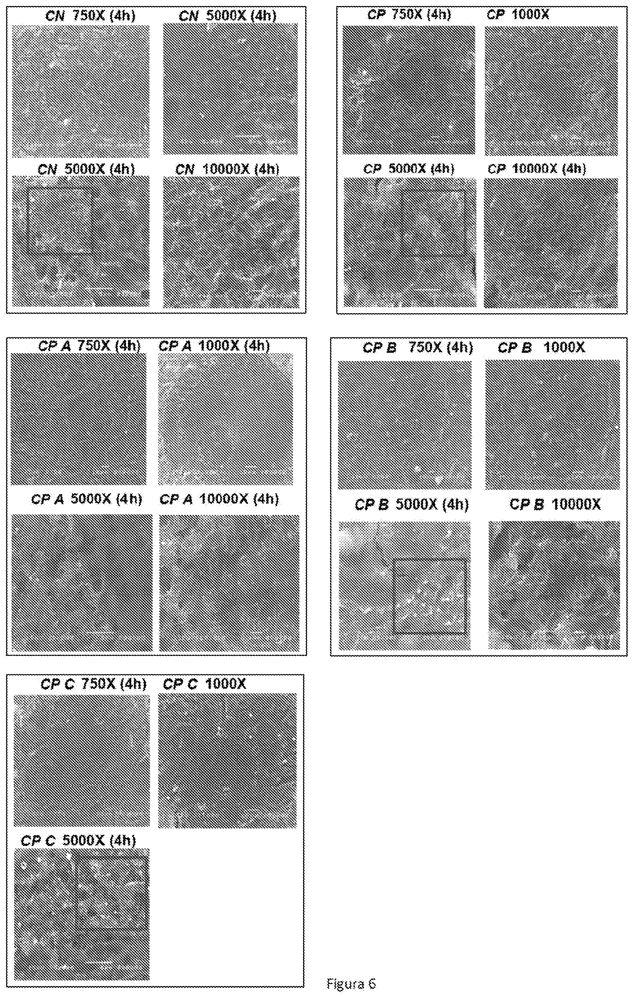

Figure 6 shows the results obtained through said SEM microscopy after pretreatment with the products A/B/C, where A=2 mM sodium butyrate, B=400 µg/ml lactoferrin, and C=2 mM sodium butyrate + 400 µg/ml lactoferrin, compared to the corresponding reference samples, NC and PC.

In the case of treatment with sodium butyrate alone (PC A), the microvilli distribution on the Caco-2 surface appears less dense (magnification 10000x). The microvilli are decreased in number and appear flat on the cellular surface. The presence of few microvillis, rarely gathered in bundles, are a symptom of an incipient or poor microvillar regrowth. Therefore, the pretreatment with sodium butyrate did not have any effect in combating the partial villous atrophy subsequently induced. These results are fully in line with the barrier integrity data obtained (significant increase in permeability after treatment with sodium butyrate at the same time of the damage induction, and lower expression of the tight junctions' proteins).

In the case of treatment with lactoferrin alone (PC B), the intracellular junctions are very evident (magnification 750x), with an homogeneous microvilli distribution on the Caco-2 surface. The microvilli are denser, longer and gathered in bundles (magnification 5000x in the box and 10000x). The pretreatment with lactoferrin had, therefore, a positive effect on the villi functionality. Also in this case, the results are in line with the data obtained for the permeability, which it has not substantially changed compared to the negative control, highlighting the achievement of a good level of expression of the tight junctions' markers.

Finally, in the case of use of the combination of sodium butyrate and lactoferrin (PC C), the tissue appeared homogeneous, the junctions among cells were visible (750x), the microvilli distribution on their surface appeared homogeneous, and the microvillis appeared arborescent and well structured, structured in macroaggregates (5000x red box).

The combination of sodium butyrate and lactoferrin thus protected the cells from both the damage induced by the absence of glutamine and the subsequent addition of ethanol: there was, in fact, an increase in microvillar bundle structures, dense and upright, synonym of regrowth and regeneration (visible effect at 5000x).

Therefore, these images also confirmed the previous immunofluorescence data in relation to recovery of the tight junction proteins' intensity and barrier integrity. The entire experimentation therefore demonstrates how lactoferrin and sodium butyrate, when used in combination, exhibit a surprising synergistic effect in combating the induced epithelial damage.

### Example 2: Evaluation of the Solubility of Lactoferrin in the Presence of Sodium Butyrate.

Lactoferrin has a very low conductivity: in distilled water it appears as a flocculant solution that, with forced stirring, becomes a suspension.

Various solutions, with increasing ratios between sodium butyrate amount and lactoferrin amount, were prepared to verify the possible effect of sodium butyrate addition on lactoferrin solubility in aqueous solution.

In particular, ten different mixtures were prepared, whose weight rations are set out in the following Table 1, and the presence of undissolved body of lactoferrin was observed as a function of the increasing amount of sodium butyrate added to the solution.

As evident from the data shown in table 1, already starting from a weight ratio between sodium butyrate and lactoferrin equal to about 2, the solution became completely clear. Solutions containing equal amounts of lactoferrin, not added with sodium butyrate, showed instead the lack of dissolution of the same, which remained as a completely undissolved body in suspension

**Table 1.**

| Sample No. | Sodium butyrate/lactoferrin weight ratio | Presence of undissolved lactoferrin |
|---|---|---|
| 1 | 9.0 | no |
| 2 | 4.0 | no |
| 3 | 2.3 | no |
| 4 | 2.0 | no |
| 5 | 1.5 | yes |
| 6 | 1.0 | yes |
| 7 | 0.7 | yes |
| 8 | 0.4 | yes |
| 9 | 0.3 | yes |
| 10 | 0.1 | yes |

It is therefore evident that combining sodium butyrate with lactoferrin, in addition to producing apparent and surprising synergistic properties in the treatment of intestinal damage prevention, also entails the advantage of making lactoferrin more easily soluble in an aqueous solution, and therefore easier to formulate the respective pharmaceutical forms containing said composition for oral administration.

## Claims

1. A composition comprising lactoferrin and a salt of butyric acid, wherein the salt of butyric acid is sodium butyrate and wherein the weight ratio between sodium butyrate amount and lactoferrin amount is greater than or equal to 2.

2. The composition according to claim 1, wherein the weight ratio between sodium butyrate amount and lactoferrin amount is in the range of 2 to 5.

3. A pharmaceutical form comprising the composition according to claim 1 or 2 and at least a pharmaceutically acceptable excipient suitable for its formulation.

4. The pharmaceutical form according to claim 3, wherein said pharmaceutical form comprises a unitary dosage of sodium butyrate in the range of 2 mg to 5 g.

5. The pharmaceutical form according to claim 4, wherein said pharmaceutical form comprises a unitary dosage of sodium butyrate in an amount in the range of 10 mg to 1 g.

6. The pharmaceutical form according to claim 5, wherein said pharmaceutical form comprises a unitary dosage of sodium butyrate in the range of 450 mg to 750 mg.

7. The pharmaceutical form according to any one of claims 3 to 6, wherein said pharmaceutical form comprises lactoferrin in a unitary dosage in the range of 5 mg to 5 g, preferably in the range of 100 mg to 2000 mg, more preferably in the range of 150 mg to 350 mg.

8. The pharmaceutical form according to any one of claims 3 to 7, wherein said pharmaceutical form comprises a unitary dosage of sodium butyrate in the range of 10 mg to 1 g and a unitary dosage of lactoferrin in the range of 100 mg to 2000 mg, preferably a unitary dosage of sodium butyrate in the range of 450 mg to 750 mg and a unitary dosage of lactoferrin in the range of 150 mg to 350 mg.

9. The pharmaceutical form according to any one of claims 3 to 8, wherein said pharmaceutical form is for oral use, being said pharmaceutical form selected from the group consisting of a granulate, a powder, a syrup, a tablet, a capsule, a gel, an aqueous suspension, and a liquid.

10. The pharmaceutical form according to any one of claims 3 to 9, wherein said pharmaceutical form is in the form of a sachet, preferably a single-dose sachet.

11. A composition comprising lactoferrin and sodium butyrate for use in the prevention of intestinal villous atrophy typical of the elderly patients, wherein the weight ratio between sodium butyrate amount and lactoferrin amount is greater than or equal to 2.

12. The composition according to claim 11, wherein the weight ratio between sodium butyrate amount and lactoferrin amount is in the range of 2 to 5.

## Patentansprüche

1. Zusammensetzung, umfassend Lactoferrin und ein Salz der Buttersäure, wobei das Salz der Buttersäure Natriumbutyrat ist und wobei das Gewichtsverhältnis zwischen Natriumbutyratmenge und Lactoferrinmenge größer oder gleich 2 ist.

2. Zusammensetzung nach Anspruch 1, wobei das Gewichtsverhältnis zwischen Natriumbutyratmenge und Lactoferrinmenge im Bereich von 2 bis 5 liegt.

3. Pharmazeutische Form, umfassend die Zusammensetzung nach Anspruch 1 oder 2 und mindestens einen pharmazeutisch verträglichen Exzipienten, der für ihre Formulierung geeignet ist.

4. Pharmazeutische Form nach Anspruch 3, wobei die pharmazeutische Form eine einheitliche Dosierung von Natriumbutyrat im Bereich von 2 mg bis 5 g umfasst.

5. Pharmazeutische Form nach Anspruch 4, wobei die pharmazeutische Form eine einheitliche Dosierung von Natriumbutyrat in einer Menge im Bereich von 10 mg bis 1 g umfasst.

6. Pharmazeutische Form nach Anspruch 5, wobei die pharmazeutische Form eine einheitliche Dosierung von Natriumbutyrat im Bereich von 450 mg bis 750 mg umfasst.

7. Pharmazeutische Form nach einem der Ansprüche 3 bis 6, wobei die pharmazeutische Form Lactoferrin in einer einheitlichen Dosierung im Bereich von 5 mg bis 5 g, vorzugsweise im Bereich von 100 mg bis 2000 mg, bevorzugter im Bereich von 150 mg bis 350 mg umfasst.

8. Pharmazeutische Form nach einem der Ansprüche 3 bis 7, wobei die pharmazeutische Form eine einheitliche Dosierung von Natriumbutyrat im Bereich von 10 mg bis 1 g und eine einheitliche Dosierung von Lactoferrin im Bereich von 100 mg bis 2000 mg, vorzugsweise eine einheitliche Dosierung von Natriumbutyrat im Bereich von 450 mg bis 750 mg und eine einheitliche Dosierung von Lactoferrin im Bereich von 150 mg bis 350 mg umfasst.

9. Pharmazeutische Form nach einem der Ansprüche 3 bis 8, wobei die pharmazeutische Form zur oralen Verwendung bestimmt ist, wobei die pharmazeutische Form aus der Gruppe ausgewählt ist, bestehend aus einem Granulat, einem Pulver, einem Sirup, einer Tablette, einer Kapsel, einem Gel, einer wässrigen Suspension und einer Flüssigkeit.

10. Pharmazeutische Form nach einem der Ansprüche 3 bis 9, wobei die pharmazeutische Form in Form eines Beutels, vorzugsweise eines Einzeldosisbeutels, vorliegt.

11. Zusammensetzung, umfassend Lactoferrin und Natriumbutyrat zur Verwendung bei der Prävention von Darmzottenatrophie, die für ältere Patienten typisch ist, wobei das Gewichtsverhältnis zwischen Natriumbutyratmenge und Lactoferrinmenge größer oder gleich 2 ist.

12. Zusammensetzung nach Anspruch 11, wobei das Gewichtsverhältnis zwischen Natriumbutyratmenge und Lactoferrinmenge im Bereich von 2 bis 5 liegt.

## Revendications

1. Composition, comprenant de la lactoferrine et un sel d'acide butyrique, dans laquelle le sel d'acide butyrique est le butyrate de sodium et dans laquelle le rapport pondéral entre la quantité de butyrate de sodium et la quantité de lactoferrine est supérieur ou égal à 2.

2. Composition selon la revendication 1, dans laquelle le rapport pondéral entre la quantité de butyrate de sodium et la quantité de lactoferrine est compris entre 2 et 5.

3. Forme pharmaceutique, comprenant la composition selon la revendication 1 ou 2 et au moins un excipient pharmaceutiquement acceptable approprié pour sa formulation.

4. Forme pharmaceutique selon la revendication 3, dans laquelle ladite forme pharmaceutique comprend un dosage unitaire de butyrate de sodium compris entre 2 mg et 5 g.

5. Forme pharmaceutique selon la revendication 4, dans laquelle ladite forme pharmaceutique comprend un dosage unitaire de butyrate de sodium dans une quantité comprise entre 10 mg et 1 g.

6. Forme pharmaceutique selon la revendication 5, dans laquelle ladite forme pharmaceutique comprend un dosage unitaire de butyrate de sodium compris entre 450 mg et 750 mg.

7. Forme pharmaceutique selon l'une quelconque des revendications 3 à 6, dans laquelle ladite forme pharmaceutique comprend de la lactoferrine à un dosage unitaire compris entre 5 mg et 5 g, de préférence entre 100 mg et 2 000 mg, plus préférablement entre 150 mg et 350 mg.

8. Forme pharmaceutique selon l'une quelconque des revendications 3 à 7, dans laquelle ladite forme pharmaceutique comprend un dosage unitaire de butyrate de sodium compris entre 10 mg et 1 g et un dosage unitaire de lactoferrine compris entre 100 mg et 2 000 mg, de préférence un dosage unitaire de butyrate de sodium compris entre 450 mg et 750 mg et un dosage unitaire de lactoferrine compris entre 150 mg et 350 mg.

9. Forme pharmaceutique selon l'une quelconque des revendications 3 à 8, dans laquelle ladite forme pharmaceutique est à usage oral, ladite forme pharmaceutique étant choisie dans le groupe consistant en un granulé, une poudre, un sirop, un comprimé, une capsule, un gel, une suspension aqueuse et un liquide.

10. Forme pharmaceutique selon l'une quelconque des revendications 3 à 9, dans laquelle ladite forme pharmaceutique se présente sous la forme d'un sachet, de préférence un sachet monodose.

11. Composition, comprenant de la lactoferrine et du butyrate de sodium destinée à être utilisée dans la prévention de l'atrophie villositaire intestinale typique des patients âgés, dans laquelle le rapport pondéral entre la quantité de butyrate de sodium et la quantité de lactoferrine est supérieur ou égal à 2.

12. Composition selon la revendication 11, dans laquelle le rapport pondéral entre la quantité de butyrate de sodium et la quantité de lactoferrine est compris entre 2 et 5.
